(19)

Europäisches
Patentamt

European
Patent Office

Office européen
des brevets

(11) **EP 3 381 441 B1**

(12) **EUROPEAN PATENT SPECIFICATION**

(45) Date of publication and mention
of the grant of the patent:
**07.04.2021  Bulletin 2021/14**

(21) Application number: **16916307.8**

(22) Date of filing: **13.09.2016**

(51) Int Cl.:
**A61Q 19/08** *(2006.01)*      **A61K 8/9789** *(2017.01)*

(86) International application number:
**PCT/KR2016/010362**

(87) International publication number:
**WO 2018/052152 (22.03.2018 Gazette 2018/12)**

(54) **EXTERNAL USE SKIN COMPOSITION FOR ANTIAGING CONTAINING GERMINATED GLYCINE GRACILIS EXTRACT AS ACTIVE INGREDIENT**

HAUTZUSAMMENSETZUNG ZUR ÄUSSEREN VERWENDUNG GEGEN HAUTALTERUNG ENTHALTEND ALS AKTIVSTOFF EINEN EXTRAKT VON GEKEIMTER GLYCINE GRACILIS

COMPOSITION POUR LA PEAU À USAGE EXTERNE CONTENANT DE L'EXTRAIT DE GLYCINE GRACILIS GERMINÉ COMME INGRÉDIENT ACTIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.10.2018   Bulletin 2018/40**

(73) Proprietor: **Amorepacific Corporation**
**Seoul 04386 (KR)**

(72) Inventors:
• **KANG, Young Gyu**
**Yongin-si**
**Gyeonggi-do 17074 (KR)**
• **KIM, Myo Yeon**
**Yongin-si**
**Gyeonggi-do 17074 (KR)**

• **PARK, Jun Seong**
**Yongin-si**
**Gyeonggi-do 17074 (KR)**

(74) Representative: **Brevalex**
**56, Boulevard de l'Embouchure**
**B.P. 27519**
**31075 Toulouse Cedex 2 (FR)**

(56) References cited:
**EP-A1- 1 537 849       KR-A- 20120 057 020**
**KR-A- 20130 107 123     KR-A- 20140 011 583**
**KR-A- 20170 025 713     US-A1- 2004 142 007**

• **SALVADOR, VICTOR H. ET AL.: 'Cinnamic Acid Increases Lignin Production And Inhibits Soybean Root Growth' PLOS ONE vol. 8, no. 7, July 2013, pages 1 - 10, XP055482778**

EP 3 381 441 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**[Technical Field]**

The present invention relates to an external use skin preparation composition for anti-aging containing a germinated *Glycine gracilis* extract as an active ingredient. More specifically, the present invention relates to an external use skin preparation composition having excellent anti-aging effects, by containing a *Glycine gracilis* extract obtained from *Glycine gracilis* germinated in a culture medium to which cinnamic acid is added.

**[Background Art]**

The skin is a primary defense barrier of the human body, which functions to protect various organs in the body from changes in temperature and humidity, and external environmental stimuli such as UV and pollutants, and plays an important role in maintaining homeostasis *in vivo.* However, excessive physical and chemical stimuli from the external environment, stresses, nutritional deficiency and the like deteriorate the normal function of the skin and accelerate skin aging phenomena such as loss of elasticity, keratinization and wrinkle formation and the like.
To prevent such phenomena and to maintain more healthy and more elastic skin, there have been efforts to manufacture cosmetics containing physiologically active substances obtained from various plants, microorganisms and the like, so as to maintain the intrinsic function of the skin and activate skin cells, thus effectively suppressing skin aging. However, most of the existing cosmetic raw materials have various problems such as exhibiting insufficient effectiveness or inducing skin side effects.
As used herein, *Glycine gracilis* is derived from the word origin "having a flat shape", and is variously referred to as nap-ddae-gi beans, nap-jjo-re-gi beans (Cheongsong), nap-deu-re beans (Young il), nap-jak beans (Gyeonggi province) and the like. Unlike soybeans, which are the most common beans, it is elliptical and has a flat and slender shape. When the nap-ddae-gi beans are planted before Chobok (one of the hottest day of the summer), the vines are extensively stretched out, but when they are planted after Chobok, they give rise to a short stalk, and the branch is fully loaded with beans such that not even a foot can be stepped into the bottom of the head of the nap-ddae-gi beans. The leaves of the nap-ddae-gi beans are relatively narrow and small and bloom blue flowers. When the nap-ddae-gi beans are raised as bean sprouts, the growth is rapid, the head part is slim, small and soft, and they have good taste.
Based on the characteristics that beans are rich in antioxidant and anti-aging components, the present inventors have found in Korean Patent Laid-Open Publication NO. 2013-0107123 that the composition containing an extract of nap-ddae-gi beans (*Glycine gracilis*) has excellent antioxidant, anti-aging, whitening or moisturizing effects.
As an extension of research on the anti-aging effects of such a *Glycine gracilis* extract, the present inventors have made extensive efforts to further enhance the effect of the *Glycine gracilis* extract in improving skin wrinkles and promoting skin elasticity, and as a result, it was found that a germinated *Glycine gracilis* extract, especially, a *Glycine gracilis* extract germinated from a culture medium to which cinnamic acid is added, further promoted the expression of dermal fibroblast growth factor and further inhibited the expression of collagenase, thus exhibiting excellent inhibitory effects on skin aging compared to a non-germinated *Glycine gracilis* extract or a *Glycine gracilis* extract germinated without cinnamic acid, thereby completing the present invention.

[Prior Art Literature]

[Patent Literature]

1. Korean Patent Laid-Open Publication No. 2013-0107123 (2013.10.01)

**[DETAILED DESCRIPTION OF THE INVENTION]**

**[Technical Problem]**

It is one object of the present invention to provide an external use skin preparation composition containing a germinated *Glycine gracilis* extract as an active ingredient, thereby exhibiting excellent anti-aging effects.

**[Technical Solution]**

In order to achieve the object above, the present invention provides an external use skin preparation composition for anti-aging comprising a germinated *Glycine gracilis* extract as an active ingredient, wherein the *Glycine gracilis* extract is obtained from *Glycine gracilis* germinated in a culture medium to which cinnamic acid is added.

The present invention also provides an external use skin preparation composition for improving skin wrinkles comprising a germinated *Glycine gracilis* extract as an active ingredient, wherein the *Glycine gracilis* extract is obtained from *Glycine gracilis* germinated in a culture medium to which cinnamic acid is added.

In addition, the present invention provides an external use skin preparation composition for improving skin elasticity comprising a germinated *Glycine gracilis* extract as an active ingredient, wherein the *Glycine gracilis* extract is obtained from *Glycine gracilis* germinated in a culture medium to which cinnamic acid is added.

Further, the present invention provides use of a germinated *Glycine gracilis* extract as an agent for improving skin wrinkles in the preparation of an external use skin preparation composition (in particular, a cosmetic composition or a pharmaceutical composition).

Further, the present invention provides use of a germinated *Glycine gracilis* extract as an agent for improving skin elasticity in the preparation of an external use skin preparation composition (in particular, a cosmetic composition or a pharmaceutical composition).

[ADVANTAGEOUS EFFECTS]

The use of the external use skin preparation composition according to the present invention increases the expression of dermal fibroblast growth factor of dermal fibroblasts synthesizing collagen, inhibits the expression and activity of collagenase, and thereby provides excellent anti-aging effects in enhancing skin elasticity and improving wrinkles.

[BRIEF DESCRIPTION OF DRAWINGS]

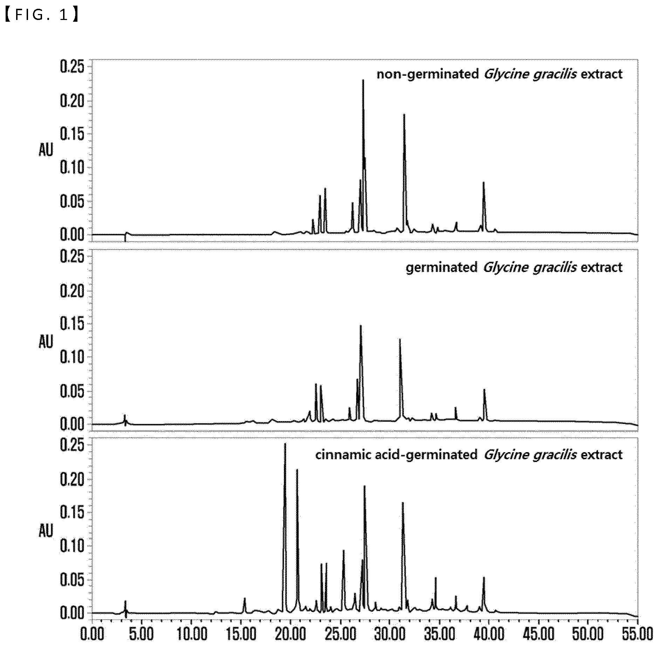

FIG. 1 shows the results of the component analysis on each of the non-germinated *Glycine gracilis* extract (Comparative Example 1), germinated *Glycine gracilis* extract (Comparative Example 2), and cinnamic acid-germinated *Glycine gracilis* extract (Example 1) using HPLC (high performance liquid chromatography).

[DETAILED DESCRIPTION OF THE EMBODIMENTS]

Unless otherwise defined, all technical and scientific terms used herein have the same meaning as commonly understood by one of ordinary skill in the art to which the present invention belongs. In general, the nomenclatures used herein are those well-known and commonly used in the art.

As used herein, the "cinnamic acid-germinated *Glycine gracilis* extract" means a substance extracted from *Glycine gracilis* germinated in a culture medium to which cinnamic acid is added, and the "germinated *Glycine gracilis* extract" without the prefix "cinnamic acid" means a substance extracted from *Glycine gracilis* germinated in an ordinary culture medium to which cinnamic acid is not added.

Further, as used herein, "collagen degrading enzyme" and "collagenase" are used as having the same meaning.

In embodiments of the present invention, the expressions of the dermal fibroblast growth factor and collagenase of the extract obtained from *Glycine gracilis* germinated from a cinnamic acid aqueous solution, the extract obtained from *Glycine gracilis* germinated without the cinnamic acid aqueous solution, and the extract obtained from non-germinated *Glycine gracilis* were each measured and compared. As a result, it was found that the *Glycine gracilis* extract germinated from the cinnamic acid aqueous solution further promotes the expression of the dermal fibroblast growth factor, inhibits the expression and activity of collagenase, and thereby exhibits excellent skin wrinkle-improving effect and skin elasticity-promoting effect.

Therefore, in one aspect, the present invention relates to an external use skin preparation composition for anti-aging comprising a germinated *Glycine gracilis* extract as an active ingredient, wherein the *Glycine gracilis* extract is obtained from *Glycine gracilis* germinated in a culture medium to which cinnamic acid is added.

In another aspect, the present invention relates to an external use skin preparation composition for improving skin wrinkles or improving skin elasticity comprising a germinated *Glycine gracilis* extract as an active ingredient, wherein the *Glycine gracilis* extract is obtained from *Glycine gracilis* germinated in a culture medium to which cinnamic acid is added.

In the present invention, the composition has a feature in that it exhibits anti-aging effects, especially, the skin elasticity-improving effect and skin wrinkle-improving effect, by promoting the expression of dermal fibroblast growth factor synthesizing collagen, which is the major constituent of the skin tissue, or inhibiting the expression of collagenase.

The scientific name for Nap-jak beans used herein is *Glycine gracilis,* which is variously referred to as nap-ddae-gi beans, nap-dae-gi beans, nap-jjo-re-gi beans, nap-deu-re beans, nap-jap beans and the like in Korean. The term "germination" refers to a phenomenon in which sprouts/shoots/buds formed on seeds, spores, pollens, and branches or roots, and the like of a plant start to develop or grow, and the term "germinated *Glycine gracilis*" means *Glycine gracilis* whose sprouts/shoots have begun to develop. In the present invention, the *Glycine gracilis* whose sprouts/shoots have grown about 20 mm to 60 mm was defined as "germinated *Glycine gracilis*".

In addition, in the present invention, the cinnamic acid used for germinating *Glycine gracilis* is also known as sinnamsan(in Korean), yukgyesan(in Korean), β-phenybcryhc acid, and is one of the typical aromatic unsaturated carboxylic acids. It is abundant in vegetable/plant extracts such as cinnamon oil, Peru balsam and oriental sweetgum oil, and it can be synthesized artificially. In the present invention, it is presumed that the cinnamic acid added to the culture medium has a function similar to that of a plant hormone, thereby inducing signaling system in the plant to produce more anti-aging-related substances. It is most preferred that the cinnamic acid is added to the culture medium, but jasmonic acid or p-coumaric acid may be used alternatively.

In the present invention, the cinnamic acid-added culture medium used for germination of *Glycine gracilis* may refer to as a cinnamic acid aqueous solution, or it may further contain an auxiliary component which can be commonly added to the culture solution of the cinnamic acid aqueous solution. The concentration of cinnamic acid in the cinnamic acid - added culture medium may preferably be 0.50 mM to 1.00 mM, more preferably 0.75 mM.

In the present invention, the *Glycine gracilis* extract may be prepared by a process comprising:

a) germinating *Glycine gracilis* using a culture medium to which cinnamic acid is added;
b) drying the *Glycine gracilis* germinated in step a), and then pulverizing the same;
c) subjecting the *Glycine gracilis* powder pulverized in step b) to a primary extraction by boiling after mixing with a polar solvent;
d) filtering the primary extract obtained in step c);
e) subjecting the extract filtered in step d) to a secondary extraction by a low-polar or non-polar organic solvent; and
f) concentrating the final extract obtained in step e) under reduced pressure.

The cinnamic acid-germinated *Glycine gracilis* extract contained in the composition of the present invention is particularly prepared by a sequential extraction by the change in polarity. The cinnamic acid-germinated *Glycine gracilis* extract is subjected to a primary extraction by a polar solvent such as an anhydrous or a functional lower alcohol (having 1 to 3 carbon atoms), acetone or the like, preferably ethanol, followed by a secondary extraction by a low-polar or non-polar solvent such as ethyl acetate, diethyl ether, benzene, chloroform, hexane or the like, preferably ethyl acetate, to maximize the content of the active ingredients and remove impurities, thereby preparing an extract having improved stability and maximized efficacy. Then, the composition can be used by adding it to an external use skin preparation, including a softening cosmetic water using a solubilizing technique, a nourishing lotion, cream, essence or the like using emulsifying technique.

In addition, the extract can be effectively purified by carrying out a filtration process twice between the primary extraction using the polar solvent and the secondary extraction. It is preferred that the primary extraction is carried out by a 200 to 300-mesh filter cloth, and the filtrate obtained by the primary filtration is allowed to age at 4°C to 15°C, followed by carrying out a secondary filtration by a filter paper.

Further, in the present invention, the cinnamic acid-germinated *Glycine gracilis* extract may be contained in an amount of 0.01% by weight to 10% by weight based on the total weight of the composition. If the content of the extract is less than 0.01% by weight, the effect may not be easily expected. If the content exceeds 10% by weight, the increase in the effect may no longer be apparent.

When the external use skin preparation composition according to the present invention is formulated in the form of a cosmetic, it may be formulated in the form of a softening cosmetic water, astringent cosmetic water, nourishing cosmetic water, eye cream, nourishing cream, massage cream, cleansing cream, cleansing foam, cleansing water, powder, essence, or facial pack, and the formulation thereof is not particularly limited. The composition according to the present invention may contain additives which are conventionally used in the cosmetic field or the skin science field including fatty substances, organic solvents, solubilizing agents, thickeners, gelling agents, softeners, antioxidants, suspending agents, stabilizers, foaming agents, fragrance ingredients, surfactants, water, ionic emulsifying agents or non-ionic emulsifying agents, fillers, sequestering agents, chelating agents, preservatives, vitamins, blockers, wetting agents, essential oils, dyes, pigments, hydrophilic activators or hydrophobic activators, lipid vesicles or any other components commonly used in cosmetics. These additives are contained in amounts which are generally used in the cosmetic field or the skin science field. Further, the composition of the present invention may contain a skin absorption promoting substance to increase the skin-improving effect.

In addition to the above-mentioned substances, the composition according to the present invention may include other components within a range that does not impair the main effect, preferably which can exert a synergistic effect on the main effect. The external use skin preparation composition according to the present invention may further include moisturizers, emollients, ultraviolet absorbers, preservatives, disinfectants, antioxidants, pH adjusters, organic dyes or inorganic dyes, flavoring agents, cooling agents or antiperspirants. The mixing amount of the above components can be easily selected by a person skilled in the art within a range that does not impair the objects and effects of the present invention, and the mixing amount may be 0.01% by weight to 5% by weight, specifically, 0.01% by weight to 3% by weight based on the total weight of the composition.

**[DETAILED DESCRIPTION OF THE EMBODIMENTS]**

Hereinafter, the present invention will be described in detail by way of Examples. However, it will be apparent to those skilled in the art that these Examples are given for illustrative purposes only, and are not intended to limit the scope of the invention thereto. Therefore, the substantial scope of the present invention will be defined by the accompanying claims and their equivalents.

**[Example 1]** Preparation of cinnamic acid-germinated *Glycine gracilis* extract

100 g of *Glycine gracilis* was germinated for 48 hours in a germinator maintaining at a temperature of 25°C using water added with cinnamic acid at a concentration of 0.75 mM. The average length of *Glycine gracilis* sprouts germinated for 48 hours was 25 mm.
Then, the germinated *Glycine gracilis* was washed with purified water, dried and pulverized to obtain 100 g of cinnamic acid germinated *Glycine gracilis* powder. 100 g of the cinnamic acid germinated *Glycine gracilis* powder was added to 1 liter of a 70% ethanol aqueous solution, and the mixture was extracted by boiling for 12 hours in an extractor equipped with a cooling condenser, and then filtered through a 300-mesh filter cloth. The filtrate thus obtained was aged by allowing to stand at 4°C to 15°C for 7 days and filtered once again with a Whatman #2 filter paper. The extract thus obtained was poured into a separatory funnel, and 1 liter of ethyl acetate was added thereto. Then, the mixture was shaken and mixed thoroughly, and when the two layers were completely separated, the upper layer (ethyl acetate layer) was collected. The lower layer (water layer) was extracted by repeating the above procedure twice using a separatory funnel. The upper layers separated in each of the extraction processes were combined, and then concentrated under reduced pressure at 50°C using a distillation apparatus equipped with a cooling condenser and dried to obtain a cinnamic acid germinated *Glycine gracilis* extract (dry weight 13.54 g).

**[Comparative Example 1]** Preparation of non-germinated *Glycine gracilis* extract

Non-germinated *Glycine gracilis* was washed with purified water, dried and pulverized to obtain 100 g of *Glycine gracilis* powder. 100 g of the *Glycine gracilis* powder was added to 1 liter of a 70% ethanol aqueous solution, and the mixture was extracted by boiling for 12 hours in an extractor equipped with a cooling condenser, and then filtered through a 300-mesh filter cloth. The filtrate thus obtained was aged by allowing to stand at 4°C to 15°C for 7 days and filtered once again with a Whatman #2 filter paper. The extract thus obtained was poured into a separatory funnel, and 1 liter of ethyl acetate was added thereto. Then, the mixture was shaken and mixed thoroughly, and when the two layers were completely separated, the upper layer (ethyl acetate layer) was collected. The lower layer (water layer) was extracted by repeating the above procedure twice using a separatory funnel. The upper layers separated in each of the extraction processes were combined, and then concentrated under reduced pressure at 50°C using a distillation apparatus equipped with a cooling condenser and dried to obtain a non-germinated *Glycine gracilis* extract (dry weight 15.18 g).

**[Comparative Example 2]** Preparation of germinated *Glycine gracilis* extract

First, 100 g of *Glycine gracilis* was germinated for 48 hours in a germinator which maintains a temperature of 25°C using primary distilled water. The average length of *Glycine gracilis* sprouts germinated for 48 hours was 55 mm. The reason why the average length of the *Glycine gracilis* sprouts is longer compared to those germinated using cinnamic acid in Example 1 is because the cinnamic acid inhibited the length of root growth in soybeans *(see,* http://journals.plos.org/plosone/article?id=10.1371/journal.pone.0069105).
Then, the germinated *Glycine gracilis* was washed with purified water, dried and pulverized to obtain 100 g of germinated *Glycine gracilis* powder. 100 g of the germinated *Glycine gracilis* powder was added to 1 liter of a 70% ethanol aqueous solution, and the mixture was extracted by boiling for 12 hours in an extractor equipped with a cooling condenser, and then filtered through a 300-mesh filter cloth. The filtrate thus obtained was aged by allowing to stand at 4°C to 15°C for 7 days and filtered once again with a Whatman #2 filter paper. The extract thus obtained was poured into a separatory funnel, and 1 liter of ethyl acetate was added thereto. Then, the mixture was shaken and mixed thoroughly, and when the two layers were completely separated, the upper layer (ethyl acetate layer) was collected. The lower layer (water layer) was extracted by repeating the above procedure twice using a separatory funnel. The upper layers separated in each of the extraction processes were combined, and then concentrated under reduced pressure at 50°C using a distillation apparatus equipped with a cooling condenser and dried to obtain a germinated *Glycine gracilis* extract (dry weight 14.16g).

**[Test Example 1]** Comparative analysis of extract components

The components of the cinnamic acid-germinated *Glycine gracilis* extract of Example 1, the non-germinated *Glycine gracilis* extract of Comparative Example 1, and the germinated *Glycine gracilis* extract of Comparative Example 2 were each analyzed. Each extract was dissolved in 70% ethanol to prepare a solution having a concentration of 10,000 ppm, and the components were analyzed (manufactured by Waters, 2996 PDA detector) using HPLC (manufactured by Waters, 2695 model). A Mightysil RP-18 GP 250-4.6 (5μm) column manufactured by Kanto Chemical was used as the stationary phase, and the composition ratio shown in Table 1 below was used as the mobile phase.

[Table 1]

| Time (minutes) | A: Water | B: Acetonitrile |
|---|---|---|
| 0 | 95 | 5 |
| 35 | 65 | 35 |
| 45 | 5 | 95 |
| 50 | 95 | 5 |
| 55 | 95 | 5 |

As a result, as shown in FIG. 1, in the cinnamic acid-germinated *Glycine gracilis* extract, a new peak was observed, which was not observed in the non-germinated *Glycine gracilis* extract and the germinated *Glycine gracilis* extract, confirming the presence of a novel component. Therefore, it can be predicted that the cinnamic acid germinated *Glycine gracilis* extract has excellent anti-aging effects compared to the non-germinated *Glycine gracilis* extract and the germinated *Glycine gracilis* extract.

**[Test Example 2]** Experiment of measuring expression of dermal fibroblast growth factor

The expression level of dermal fibroblast growth factor in the cinnamic acid germinated *Glycine gracilis* extract of Example 1, the non-germinated *Glycine gracilis* extract of Comparative Example 1, and the germinated *Glycine gracilis* extract of Comparative Example 2 was each measured by comparing with DMEM (Dulbecco's Modified Eagle's Media) medium containing 2.5% by weight fetal bovine serum.

Human fibroblasts (manufactured by Cascade Biologics) (Portland, OR, USA) were seeded at 5,000 cells/well in a 96-well microtiter plate containing DMEM medium containing 2.5% by weight of fetal bovine serum, and cultured until it grew to about 90%. Thereafter, the cells were cultured in serum-free DMEM for 24 hours, and then treated with each of the cinnamic acid germinated *Glycine gracilis* extract of Example 1, the non-germinated *Glycine gracilis* extract of Comparative Example 1, and the germinated *Glycine gracilis* extract of Comparative Example 2 dissolved in the serum-free DMEM at a concentration of 50 ppm for 24 hours. For the positive control group, the serum-free DMEM was replaced with DMEM medium containing 2.5% by weight of fetal bovine serum, and the cells were cultured for 24 hours therein, then the cell culture medium was collected.

Subsequently, the expression level of dermal fibroblast growth factor in the collected cell culture medium was measured using a dermal fibroblast growth factor measurement device (R&D Systems, USA). First, the cell culture medium collected was added to a 96-well plate uniformly coated with primary antibodies for dermal fibroblast growth factor, and the antigen-antibody reaction was carried out in a thermostat(36°C) for 3 hours.

After 4 hours, secondary antibodies for the dermal fibroblast growth factor bound to a chromophore was added to the 96-well plate and reacted for another 15 minutes. After 15 minutes, a color development-inducing substance was added to carry out a color development at room temperature for 15 minutes, and when 1 M sulfuric acid was added again to stop the reaction (color development), the color of the reaction solution appeared yellow, and it was confirmed that the degree of blue color was different depending on the progress of the reaction.

The absorbance of the 96-well plate appearing in blue was measured at 450 nm using an absorptiometer, and the expression level of dermal fibroblast growth factor was calculated by Calculation Equation 1 below. Herein, the reaction absorbance of the cell culture medium collected from the group in which no composition was treated was used as a control. That is, the expression level of the dermal fibroblast growth factor in the untreated group was set to 100, and the expression level of dermal fibroblast growth factor in the groups treated with the test substances was determined relatively. The results are shown in Table 2 below.

[Calculation Equation 1]

$$\text{Expression level of dermal fibroblast growth factor (\%)} = \frac{\text{Absorbance of test substance-treated cell group}}{\text{Absorbance of control group}} \times 100$$

[Table 2]

| Test substances | Expression level of dermal fibroblast growth factor (%) |
|---|---|
| Untreated group | 100 |
| DMEM medium containing 2.5% by weight of fetal bovine serum | 196 |
| Cinnamic acid-germinated *Glycine gracilis* extract (Example 1) | 189 |
| Non-germinated *Glycine gracilis* extract (Comparative Example 1) | 124 |
| Germinated *Glycine gracilis* extract (Comparative Example 2) | 129 |

As shown in Table 2, it was found that the cinnamic acid-germinated *Glycine gracilis* extract of Example 1 more effectively increased the expression of dermal fibroblast growth factor *in vitro* compared to the non-germinated *Glycine gracilis* extract of Comparative Example 1 and the germinated *Glycine gracilis* extract of Comparative Example 2. Accordingly, it was confirmed that the extract of the present invention or the composition containing the same exhibits excellent anti-aging effects, that is, excellent skin wrinkle-improving effect and skin elasticity-improving effect.

[Test Example 3] Anti-aging potency test

The inhibitory ability of the cinnamic acid-germinated *Glycine gracilis* extract of Example 1, the non-germinated *Glycine gracilis* extract of Comparative Example 1, and the germinated *Glycine gracilis* extract of Comparative Example 2 on the collagenase production was each measured by comparison with tocopherol and EGCG. The tocopherol and EGCG are substances known to function in preventing skin aging by regenerating epidermal cells of the skin.

Human fibroblasts (manufactured by Cascade Biologics) (Portland, OR, USA) were seeded at 5,000 cells/well in a 96-well microtiter plate containing DMEM medium containing 2.5% by weight of fetal bovine serum, and cultured until it grew to about 90%. Thereafter, the cells were cultured in serum-free DMEM for 24 hours, then treated with each of the cinnamic acid germinated *Glycine gracilis* extract of Example 1, the non-germinated *Glycine gracilis* extract of Comparative Example 1, and the germinated *Glycine gracilis* extract of Comparative Example 2 dissolved in the serum-free DMEM at a concentration of 50 ppm for 24 hours and treated with tocopherol and EGCG (manufactured by Sigma Aldrich) at a concentration of $50\mu$M for 24 hours. And, then the cell culture medium was collected.

Subsequently, the production level of collagenase in the collected cell culture medium was measured using a collagenase measurement device (Amersham Pharmacia, USA). First, the cell culture medium collected was added to a 96-well plate uniformly coated with primary collagenase antibodies, and the antigen-antibody reaction was carried out in a thermostat(36°C) for 3 hours.

After 3 hours, secondary collagenase antibodies bound to a chromophore was added to the 96-well plate and reacted for another 15 minutes. After 15 minutes, a color development-inducing substance was added to carry out a color development at room temperature for 15 minutes, and when 1 M sulfuric acid was added again to stop the reaction (color development), the color of the reaction solution appeared yellow, and it was confirmed that the degree of yellow color was different depending on the progress of the reaction.

The absorbance of the 96-well plate appearing in yellow was measured at 450 nm using an absorptiometer, and the degree of collagenase synthesis was calculated by Calculation Equation 2 below. Herein, the reaction absorbance of the cell culture medium collected from the group in which no composition was treated was used as a control. That is, the expression level of collagenase in the untreated group was set to 100, and the expression level of collagenase in the groups treated with the test substances was determined relatively. The results are shown in Table 3 below.

**[Calculation Equation 2]**

$$\text{Expression level of collagenase (\%)} = \frac{\text{Absorbance of test substance-treated cell group}}{\text{Absorbance of control group}} \times 100$$

[Table 3]

| Test substances | Expression level of collagenase (%) |
|---|---|
| Untreated group | 100 |
| Tocopherol | 75 |
| EGCG | 61 |
| Cinnamic acid germinated *Glycine gracilis* extract (Example 1) | 58 |
| Non-germinated *Glycine gracilis* extract (Comparative Example 1) | 79 |
| Germinated *Glycine gracilis* extract (Comparative Example 2) | 76 |

As shown in Table 3, it was found that the cinnamic acid-germinated *Glycine gracilis* extract of Example 1 more effectively inhibited the expression of collagenase *in vitro* compared to the non-germinated *Glycine gracilis* extract of Comparative Example 1 and the germinated *Glycine gracilis* extract of Comparative Example 2 and that it had a superior inhibitory ability on the expression of collagenase compared to tocopherol and EGCG, which are known as anti-aging substances. Accordingly, it was confirmed that the extract of the present invention or the composition containing the same exhibits excellent anti-aging effects, that is, excellent skin wrinkle-improving effect and skin elasticity-improving effect.

Although specific parts of the present invention have been described in detail, it will be apparent to those skilled in the art that these specific techniques are merely a preferred embodiment and that the scope of the present invention is not limited thereto. Therefore, the substantial scope of the present invention will be defined by the accompanying claims and their equivalents.

In addition, Formulation Examples of external use skin preparation compositions including the composition of the present invention will be described in more detail below. However, it will be apparent that the scope of the present invention is not limited thereto.

**[Formulation Example 1]** Nourishing cosmetic water

A nourishing cosmetic water was prepared in a conventional manner according to the composition shown in Table 4 below (unit: wt%).

[Table 4]

| Ingredients | Amount |
|---|---|
| Purified water | Residual amount |
| Glycerin | 8.0 |
| Butylene glycol | 4.0 |
| Hyaluronic acid extract | 5.0 |

(continued)

| Ingredients | Amount |
|---|---|
| Beta glucan | 7.0 |
| Carbomer | 0.1 |
| Cinnamic acid germinated *Glycine gracilis* extract (Example 1) | 0.05 |
| Caprylic / capric triglyceride | 8.0 |
| Squalene | 5.0 |
| Cetearyl glucoside | 1.5 |
| Sorbitan stearate | 0.4 |
| Cetearyl alcohol | 1.0 |
| Triethanolamine | 0.1 |

**[Formulation Example 2]** Nourishing cream

A nourishing cream was prepared in a conventional manner according to the composition shown in Table 5 below (unit: wt%).

[Table 5]

| Ingredients | Amount |
|---|---|
| Purified water | Residual amount |
| Glycerin | 3.0 |
| Butylene glycol | 3.0 |
| Liquid paraffin | 7.0 |
| Beta glucan | 7.0 |
| Carbomer | 0.1 |
| Cinnamic acid germinated *Glycine gracilis* extract (Example 1) | 3.0 |
| Caprylic / capric triglyceride | 3.0 |
| Squalene | 5.0 |
| Cetearyl glucoside | 1.5 |
| Sorbitan stearate | 0.4 |
| Polysorbate 60 | 1.2 |
| Triethanolamine | 0.1 |

**[Formulation Example 3]** Massage cream

A massage cream was prepared in a conventional manner according to the composition shown in Table 6 below (unit: wt%).

[Table 6]

| Ingredients | Amount |
|---|---|
| Purified water | Residual amount |
| Glycerin | 8.0 |
| Butylene glycol | 4.0 |

(continued)

| Ingredients | Amount |
|---|---|
| Liquid paraffin | 45.0 |
| Beta glucan | 7.0 |
| Carbomer | 0.1 |
| Cinnamic acid germinated *Glycine gracilis* extract (Example 1) | 1.0 |
| Caprylic / capric triglyceride | 3.0 |
| Beeswax | 4.0 |
| Cetearyl glucoside | 1.5 |
| Sorbitan Sesquioleate | 0.9 |
| Vaseline | 3.0 |
| Paraffin | 1.5 |

**[Formulation Example 4]** Facial pack

A facial pack was prepared in a conventional manner according to the composition shown in Table 7 below (unit: wt%).

[Table 7]

| Ingredients | Amount |
|---|---|
| Purified water | Residual amount |
| Glycerin | 4.0 |
| Polyvinyl alcohol | 15.0 |
| Hyaluronic acid extract | 5.0 |
| Beta glucan | 7.0 |
| Allantoin | 0.1 |
| Cinnamic acid germinated *Glycine gracilis* extract (Example 1) | 0.5 |
| Nonylphenyl ether | 0.4 |
| Polysorbate 60 | 1.2 |
| Ethanol | 6.0 |

**[Formulation Example 5]** Ointment in external use skin preparation

An ointment was prepared in a conventional manner according to the composition shown in Table 8 below (unit: wt%).

[Table 8]

| Ingredients | Amount |
|---|---|
| Purified water | Residual amount |
| Glycerin | 8.0 |
| Butylene glycol | 4.0 |
| Liquid paraffin | 15.0 |
| Beta glucan | 7.0 |

(continued)

| Ingredients | Amount |
|---|---|
| Carbomer | 0.1 |
| Cinnamic acid germinated *Glycine gracilis* extract (Example 1) | 1.0 |
| Caprylic / capric triglyceride | 3.0 |
| Squalene | 1.0 |
| Cetearyl glucoside | 1.5 |
| Sorbitan stearate | 0.4 |
| Cetearyl alcohol | 1.0 |
| Beeswax | 4.0 |

**Claims**

1. An external use skin preparation composition for anti-aging comprising a germinated *Glycine gracilis* extract as an active ingredient,
   wherein the *Glycine gracilis* extract is obtained from *Glycine gracilis* germinated in a culture medium to which cinnamic acid is added.

2. An external use skin preparation composition for improving skin wrinkles comprising a germinated *Glycine gracilis* extract as an active ingredient,
   wherein the *Glycine gracilis* extract is obtained from *Glycine gracilis* germinated in a culture medium to which cinnamic acid is added.

3. An external use skin preparation composition for improving skin elasticity comprising a germinated *Glycine gracilis* extract as an active ingredient,
   wherein the *Glycine gracilis* extract is obtained from *Glycine gracilis* germinated in a culture medium to which cinnamic acid is added.

4. The external use skin preparation composition of any one of claims 1 to 3, wherein the *Glycine gracilis* extract is prepared by a process comprising the steps of:

   a) germinating *Glycine gracilis* using a culture medium to which cinnamic acid is added;
   b) drying the *Glycine gracilis* germinated in step a), and then pulverizing the same;
   c) subjecting the *Glycine gracilis* powder pulverized in step b) to a primary extraction by boiling after mixing with a polar solvent;
   d) filtering the primary extract obtained in step c);
   e) subjecting the extract filtered in step d) to a secondary extraction by a low-polar or non-polar organic solvent; and
   f) concentrating the final extract obtained in step e) under reduced pressure.

5. The external use skin preparation composition of claim 4, wherein the concentration of cinnamic acid in the cinnamic acid-added culture medium of step a) is 0.50 mM to 1.00 mM.

6. The external use skin preparation composition of claim 4, wherein the polar solvent of step c) is an alcohol having 1 to 3 carbon atoms, and the low-polar or non-polar organic solvent of step e) is ethyl acetate.

7. The external use skin preparation composition of claim 4, wherein the filtration of step d) comprises a primary filtration by a filter cloth, aging the filtrate obtained by the primary filtration at 4°C to 15°C, and a secondary filtration by a filter paper.

8. The external use skin preparation composition of any one of claims 1 to 3, wherein the germinated *Glycine gracilis* extract is contained in an amount of 0.01% by weight to 10% by weight based on the total weight of the composition.

9. The external use skin preparation composition of any one of claims 1 to 3, wherein the composition promotes the expression of a dermal fibroblast growth factor.

10. The external use skin preparation composition of any one of claims 1 to 3, wherein the composition inhibits the expression of collagenase.

11. Use of a germinated *Glycine gracilis* extract obtained from *Glycine gracilis* germinated in a culture medium to which cinnamic acid is added, as an agent for improving skin wrinkles in the preparation of an external use skin preparation composition.

12. Use of a germinated *Glycine gracilis* extract obtained from *Glycine gracilis* germinated in a culture medium to which cinnamic acid is added as an agent for improving skin elasticity in the preparation of an external use skin preparation composition.

**Patentansprüche**

1. Äußerlich anzuwendende Hautpräparat-Zusammensetzung gegen den Alterungsprozess, einen Extrakt aus gekeimter *Glycine gracilis* als Wirkstoff umfassend, wobei der *Glycine gracilis*-Extrakt aus *Glycine gracilis* erhalten wird, die in einem Kulturmedium gekeimt wurde, dem Zimtsäure zugesetzt ist.

2. Äußerlich anzuwendende Hautpräparat-Zusammensetzung zur Verbesserung von Hautfalten, einen Extrakt aus gekeimter *Glycine gracilis* als Wirkstoff umfassend, wobei der *Glycine gracilis*-Extrakt aus *Glycine gracilis* erhalten wird, die in einem Kulturmedium gekeimt wurde, dem Zimtsäure zugesetzt ist.

3. Äußerlich anzuwendende Hautpräparat-Zusammensetzung zur Verbesserung der Hautelastizität, einen Extrakt aus gekeimter *Glycine gracilis* als Wirkstoff umfassend,
wobei der *Glycine gracilis*-Extrakt aus *Glycine gracilis* erhalten wird, die in einem Kulturmedium gekeimt wurde, dem Zimtsäure zugesetzt ist.

4. Äußerlich anzuwendende Hautpräparat-Zusammensetzung nach einem der Ansprüche 1 bis 3, wobei der *Glycine gracilis*-Extrakt durch ein Verfahren hergestellt wird, welches die folgenden Schritte umfasst:

a) Keimen von *Glycine gracilis* unter Verwendung eines Kulturmediums, dem Zimtsäure zugesetzt wird,
b) Trocknen der in Schritt a) gekeimten *Glycine gracilis* und anschließendes Pulverisieren derselben,
c) Unterziehen des in Schritt b) pulverisierten *Glycine gracilis*-Pulvers einer Primärextraktion durch Sieden nach Mischen mit einem polaren Lösungsmittel,
d) Filtrieren des in Schritt c) erhaltenen Primärextrakts,
e) Unterwerfen des in Schritt d) gefilterten Extrakts einer sekundären Extraktion durch ein niederpolares oder unpolares organisches Lösungsmittel und
f) Konzentrieren des in Schritt e) erhaltenen Endextrakts unter vermindertem Druck.

5. Äußerlich anzuwendende Hautpräparat-Zusammensetzung nach Anspruch 4, wobei die Konzentration an Zimtsäure in dem mit Zimtsäure versetzten Kulturmedium von Schritt a) 0,50 mM bis 1,00 mM beträgt.

6. Äußerlich anzuwendende Hautpräparat-Zusammensetzung nach Anspruch 4, wobei das polare Lösungsmittel von Schritt c) ein Alkohol mit 1 bis 3 Kohlenstoffatomen ist und das niederpolare oder unpolare organische Lösungsmittel von Schritt e) Ethylacetat ist.

7. Äußerlich anzuwendende Hautpräparat-Zusammensetzung nach Anspruch 4, wobei die Filtration von Schritt d) eine Primärfiltration durch ein Filtertuch, Reifung des durch die Primärfiltration erhaltenen Filtrats bei 4 °C bis 15 °C und eine Sekundärfiltration durch ein Filterpapier umfasst.

8. Äußerlich anzuwendende Hautpräparat-Zusammensetzung nach einem der Ansprüche 1 bis 3, wobei der Extrakt aus gekeimter *Glycine gracilis* in einer Menge von 0,01 Gew.-% bis 10 Gew.-%, bezogen auf das Gesamtgewicht der Zusammensetzung, enthalten ist.

9. Äußerlich anzuwendende Hautpräparat-Zusammensetzung nach einem der Ansprüche 1 bis 3, wobei die Zusammensetzung die Expression eines dermalen Fibroblasten-Wachstumsfaktors fördert.

10. Äußerlich anzuwendende Hautpräparat-Zusammensetzung nach einem der Ansprüche 1 bis 3, wobei die Zusammensetzung die Expression von Kollagenase hemmt.

11. Verwendung eines Extrakts aus gekeimter *Glycine gracilis,* erhalten aus *Glycine gracilis,* gekeimt in einem Kulturmedium, dem Zimtsäure zugesetzt ist, als Mittel zur Verbesserung von Hautfalten bei der Herstellung einer äußerlich anzuwendenden Hautpräparat-Zusammensetzung.

12. Verwendung eines Extrakts aus gekeimter *Glycine gracilis,* erhalten aus *Glycine gracilis,* gekeimt in einem Kulturmedium, dem Zimtsäure zugesetzt ist, als Mittel Verbesserung der Hautelastizität bei der Herstellung einer äußerlich anzuwendenden Hautpräparat-Zusammensetzung.

**Revendications**

1. Composition pour préparation pour la peau à usage externe pour un anti-âge comprenant un extrait de *Glycine gracilis* germée en tant que principe actif,
dans laquelle l'extrait de *Glycine gracilis* est obtenu à partir de *Glycine gracilis* germée dans un milieu de culture auquel est ajouté de l'acide cinnamique.

2. Composition pour préparation pour la peau à usage externe pour améliorer les rides de la peau comprenant un extrait de *Glycine gracilis* germée en tant que principe actif,
dans laquelle l'extrait de *Glycine gracilis* est obtenu à partir de *Glycine gracilis* germée dans un milieu de culture auquel est ajouté de l'acide cinnamique.

3. Composition pour préparation pour la peau à usage externe pour améliorer l'élasticité de la peau comprenant un extrait de *Glycine gracilis* germée en tant que principe actif,
dans laquelle l'extrait de *Glycine gracilis* est obtenu à partir de *Glycine gracilis* germée dans un milieu de culture auquel est ajouté de l'acide cinnamique.

4. Composition pour préparation pour la peau à usage externe selon l'une quelconque des revendications 1 à 3, dans laquelle l'extrait de *Glycine gracilis* est préparé par un processus comprenant les étapes de :

   a) germination de *Glycine gracilis* à l'aide d'un milieu de culture auquel est ajouté de l'acide cinnamique ;
   b) séchage de la *Glycine gracilis* germée à l'étape a), puis pulvérisation de celle-ci ;
   c) soumission de la poudre de *Glycine gracilis* pulvérisée à l'étape b) à une extraction primaire par ébullition après mélange avec un solvant polaire ;
   d) filtration de l'extrait primaire obtenu à l'étape c) ;
   e) soumission de l'extrait filtré à l'étape d) à une extraction secondaire par un solvant organique faiblement polaire ou non polaire ; et
   f) concentration de l'extrait final obtenu à l'étape e) sous pression réduite.

5. Composition pour préparation pour la peau à usage externe selon la revendication 4, dans laquelle la concentration d'acide cinnamique dans le milieu de culture avec acide cinnamique ajouté de l'étape a) est de 0,50 mM à 1,00 mM.

6. Composition pour préparation pour la peau à usage externe selon la revendication 4, dans laquelle le solvant polaire de l'étape c) est un alcool ayant 1 à 3 atomes de carbone, et le solvant organique faiblement polaire ou non polaire de l'étape e) est l'acétate d'éthyle.

7. Composition pour préparation pour la peau à usage externe selon la revendication 4, dans laquelle la filtration de l'étape d) comprend une filtration primaire par un tissu filtrant, le vieillissement du filtrat obtenu par la filtration primaire de 4°C à 15°C, et une filtration secondaire par un papier filtre.

8. Composition pour préparation pour la peau à usage externe selon l'une quelconque des revendications 1 à 3, dans laquelle l'extrait de *Glycine gracilis* germée est contenu dans une quantité de 0,01% en poids à 10% en poids sur la base du poids total de la composition.

9. Composition pour préparation pour la peau à usage externe selon l'une quelconque des revendications 1 à 3, dans laquelle la composition favorise l'expression d'un facteur de croissance des fibroblastes dermiques.

10. Composition pour préparation pour la peau à usage externe selon l'une quelconque des revendications 1 à 3, dans laquelle la composition inhibe l'expression de collagénase.

11. Utilisation d'un extrait de *Glycine gracilis* germée obtenu à partir de *Glycine gracilis* germée dans un milieu de culture auquel est ajouté de l'acide cinnamique, en tant qu'agent pour améliorer les rides de la peau dans la préparation d'une composition pour préparation pour la peau à usage externe.

12. Utilisation d'un extrait de *Glycine gracilis* germée obtenu à partir de *Glycine gracilis* germée dans un milieu de culture auquel est ajouté de l'acide cinnamique, en tant qu'agent pour améliorer l'élasticité de la peau dans la préparation d'une composition pour préparation pour la peau à usage externe.

【FIG. 1】

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- NO 20130107123

- KR 20130107123